# EUROPEAN PATENT APPLICATION

(11) **EP 4 644 537 A1**
(43) Date of publication of application: **05.11.2025**
(21) Application number: 24173529.9
(22) Date of filing: 30.04.2024
(51) Int. Cl.: C12N 5/0775, A61K 39/00, C07K 14/705, C12N 15/10

(54) **TARGETED EXTRACELLULAR VESICLE**

(71) Applicant: ConvEyXo, 6041 Charleroi (BE)
(72) Inventor: ROEFS, Marieke, 1110 Vienna (AT); GRILLARI, Johannes, 1110 Vienna (AT); GRILLARI, Regina, 1110 Vienna (AT); TRAXLMAYR, Michael, 1110 Vienna (AT); BOBBILI, Madhusudhan Reddy, 1110 Vienna (AT)
(74) Representative: Calysta NV

(57) **Abstract**

An engineered Eukaryotic cell expressing a transmembrane protein having the potential to be sorted into an extracellular vesicle from the said Eukaryotic cell, wherein the extracellular domain of the said transmembrane protein comprises a segment having a high affinity for a chemical derivative not expressed by the said Eukaryotic cell, or a derivatization domain, a method to identify targeting ligands for extracellular vesicles and the extracellular vesicles decorated with such ligand.

## Description

### Technical domain

The present disclosure concerns the use of extracellular vesicles coupled to targeting moieties as a validation system, or as therapeutic agents, as well as the corresponding cell lines.

### State of the art

Extracellular vesicles (EVs) are secreted by a multitude of cell types and are typically in a size range between 30 and 500 nm of diameter. EVs are involved in several cellular processes such as intercellular communication, antigen presentation, transfer of proteins, peptides, metabolites, lipids, nucleic acids comprising messenger RNAs, siRNAs, and/or microRNAs, from one cell to another. The function of exosomes is altered in many diseases such as cancers, which suggests their importance as a diagnostic tool and/or in therapeutic applications, for example in acellular therapies as a vehicle to deliver therapeutic molecules, in immunotherapy, to treat different types of cancer and/or inflammatory and cardiovascular neurodegenerative diseases, diabetes, etc.

Some protocols for isolating and purifying EVs are known, and exosomes have already been successfully used in cell-free therapy studies.

However, the inventors do consider that there is the need to improve the specific targeting of EVs in order to change their tropism.

Several publications suggest engineering transmembrane proteins of EVs so that they express specific ligands.

Among them, the document WO2019040920 discloses a method to isolate exosomes based on a transfected producer cell where a scaffold protein further comprises a ligand, such as the F2 receptor negative regulator (PTGFRN), basigin (BSG), immunoglobulin superfamily member 2 (IGSF2), immunoglobulin superfamily member 3 (IGSF3), immunoglobulin superfamily member 8 (IGSF8), integrin beta-1 (ITGB1), ITGA4 (integrin alpha-4), 4F2 cell-surface antigen heavy chain (SLC3A2), or ATP transporter. This document favors the isolation of exosomes based on proteins scaffolds being not tetraspanins, since these proteins have both their N- and C-terminal parts located at the lumen of the exosome.

The document EP3672614 discloses extracellular vesicles where a scaffold protein further comprises a biologically active molecule at its C-terminus.

The inventors do consider that such approach is valuable, but needs a lot of time and efforts, since there is the need to engineer new genetically modified cell lines, one per ligand, and then to test the properties of the produced exosomes.

### Summary of the invention

A first aspect of the present invention is an engineered Eukaryotic cell (preferably mammalian cell) expressing an engineered transmembrane protein having the potential to be sorted into an extracellular vesicle (EV) from the said Eukaryotic cell (preferably mammalian cell),
the said engineered transmembrane protein harboring one or several extracellular domains, wherein at least one of the extracellular domain of the said engineered transmembrane protein comprises a segment having a high affinity for a chemical derivative (2) not expressed by the said Eukaryotic cell (preferably mammalian cell).

A related aspect is an extracellular vesicle obtainable from this engineered Eukaryotic cell (preferably mammalian cell), preferably wherein this Eukaryotic cell is a mammalian mesenchymal stem cell, more preferably a human mesenchymal stem cell.

Still another related aspect of the present invention is an engineered Eukaryotic cell (preferably mammalian cell) expressing an engineered transmembrane protein having the potential to be sorted into an extracellular vesicle from the said Eukaryotic cell (preferably mammalian cell), the said engineered transmembrane protein harboring one or several extracellular domains, wherein at least one of the extracellular domains of the said engineered transmembrane protein comprises a derivatization domain.

Still another related aspect of the present invention is an extracellular vesicle obtainable from this engineered Eukaryotic cell (preferably mammalian cell), preferably wherein the Eukaryotic cell (preferably mammalian cell) of is a mammalian mesenchymal stem cell, more preferably a human mesenchymal stem cell.

Preferably, in these engineered Eukaryotic cell (preferably mammalian cell) or extracellular vesicle, the engineered transmembrane protein is selected from SEQ ID NO:1 to 80 or comprises a tetraspanin (CD9, CD63, CD37, CD81, or CD82), or a functional fragment thereof, or a protein sharing at least 95% of identity over the full-length of the said SEQ ID NO:1 to 80, of the tetraspanin, or of the functional fragment thereof.

Preferably when this engineered transmembrane protein is a tetraspanin, this tetraspanin is engineered to comprise a fifth transmembrane region, such as a sequence sharing at least 50% of identity with SEQ ID NO:81, thereby advantageously allowing an extracellular C-terminal end.

Another related aspect of the present invention is a method to select a ligand 4 targeting an extracellular vesicle to a specific cell, organ, or tissue comprising the steps of:
- selecting an engineered Eukaryotic (preferably mammalian cell) cell line expressing an engineered transmembrane protein having the potential to be sorted into an extracellular vesicle from the said Eukaryotic cell (preferably mammalian cell), the said transmembrane protein comprising at least one extracellular domain, wherein at least one of the extracellular domain of the said transmembrane protein of the said engineered Eukaryotic cell (preferably mammalian cell) comprises a segment having a high affinity for a chemical derivative 2 not expressed by the said Eukaryotic cell (preferably mammalian cell);
- growing cells from the said engineered Eukaryotic cell (preferably mammalian cell) line;
- obtaining extracellular vesicles from the said grown cells and
- applying to the transmembrane protein of the obtained extracellular vesicle a bifunctional molecule 1,
   the first function being the ligand 4 to a desired target, and
   the second function being the said chemical derivative 2, so that the said chemical derivative 2 is bound with high-affinity to the said transmembrane protein and the ligand 4 is still able to recognize its desired target;
- measuring, *in vitro* and/or *in vivo,* the binding of the said extracellular vesicle to the said target.

In this method, preferably, the chemical derivative 2 is selected from the group consisting of biotin and fluorescein.

Preferably, in this method, the bifunctional molecule 1 is a peptide 3, 4 derivatized with the second function 2, preferably the peptide 3, 4 being an antibody or a fragment thereof.

Preferably, the high affinity is a dissociation constant (Kd) value of the chemical derivative 2, and/or of the second function bound to the transmembrane protein, lower than 10-9, preferably lower than 10-12 Molar (at 25°C).

Preferably, the engineered transmembrane protein is selected from SEQ ID NO:1 to 80, or comprises a tetraspanin (CD9, CD63, CD37, CD81, or CD82), or a functional fragment thereof, or a protein sharing at least 95% of identity over the full-length of the said SEQ ID NO:1 to 80, of the tetraspanin, or a functional fragment thereof.

Another related aspect of the present invention is a targeting ligand 4 obtainable by such method.

Advantageously, such method further comprises the step of grafting the identified targeting ligand 4 at the surface of Extracellular vesicles, thereby generating Extracellular vesicles with a targeting moiety for a desired target (and preferably with no engineered transmembrane protein).

Hence, possibly, this targeting ligand 4 is covalently grafted on the extracellular vesicle (preferably with no engineered transmembrane protein).

Alternatively, this targeting ligand 4 is non-covalently bound on the extracellular vesicle.

Still another option (for a targeting ligand 4 being a peptide) is an engineered Eukaryotic cell (preferably mammalian cell) transiently expressing an engineered a transmembrane protein having the potential to be sorted into an extracellular vesicle from the said Eukaryotic cell (preferably mammalian cell), the said engineered transmembrane protein comprising one or several extracellular domains, wherein at least one of the extracellular domain of the said transmembrane protein comprises this targeting ligand 4.

Another aspect of the present invention is a pharmaceutical composition comprising these grafted extracellular vesicle, or the extracellular vesicles obtained from these engineered cells upon transient transfection.

### Brief description of the Figures

Figure 1 Schematic representation of the recombinant constructs used in the examples. Figure 1a, the engineered transmembrane protein; Figure 1b, the bifunctional molecule.
Figure 2*. In vitro* validation.
Figure 3. Production and purification of EVs.
Figure 4. Correct binding of EVs on their targets
Figure 5. Constructs with other tetraspanins.

### Detailed description of the invention

The inventors have developed a system offering high flexibility to validate ligands targeting Extracellular vesicles (EVs) including *in vivo.* This system, in turn, allows to easily develop tagged EVs. In practice, the inventors have obtained higher on-target and lower off-target delivery, offering increased therapeutic windows for EVs, including for the molecules carried by them.

A first aspect of the present invention is therefore an engineered eukaryotic (plant or mammalian) cell expressing an engineered transmembrane protein, or an engineered GPI-anchor protein, having the potential to be sorted into an EV from the said eukaryotic (mammalian or plant) cell. This engineered transmembrane protein comprises at least one extracellular domain, wherein one (at least one) extracellular domain of the transmembrane protein comprises a (at least one) segment having a high affinity for a chemical derivative not expressed by the said eukaryotic (mammalian or plant) cell; or, alternatively, this GPI anchor protein, which is located at the extracellular layer of the cell membrane, comprises a segment having a high affinity for a chemical derivative not expressed by the said eukaryotic cell (plant or mammalian cell).

In the context of the present invention, "eukaryotic" refers to fungi, yeast, plant or animal cells, indeed, beside mammalian cells, other cell types can be used, including plant, fungi or yeast cells. However, mammalian are preferred cells and Human cells are more preferred cells.

In the context of the present invention, "extracellular vesicles (EVs)" preferably refer to any type of sub-micron particles surrounded by a cellular membrane and originating from a cell culture (*i.e.* a lipidic bilayer and proteins, including transmembrane proteins, such as tetraspanins).

Preferred EVs have a diameter lower than 500 nm, preferably lower than 400 nm, more preferably lower than 300 or even lower than 250 nm. Advantageously, EVs have a diameter between 30, and 200 nm.

The EVs of the present invention (originating from mammalian cells) have at least one tetraspanin present in their lipid bilayer, preferably chosen between CD9, CD63, CD81 and potentially two or the three, including peptides having at least four transmembrane domains and sharing at least 95% of identity with a sequence of at least 200 (or at least 230 or even at least 235 or 238) consecutive aminoacids starting at the N-terminal part of any of SEQ. ID NO: 87-92.

In the context of the present invention, for instance in the engineered Eukaryotic (plant, mammalian) cells or EVs thereof, the engineered transmembrane protein is preferably a tetraspanin (CD9, CD63, CD37, CD81, or CD82), preferably a human tetraspanin, or a fragment thereof. Other transmembrane proteins can advantageously be engineered (on the extracellular segment), such as those according to any one of the SEQ ID NO:1 to 80 or sharing at least 95% of identity over the full-length of any one of the SEQ ID NO:1 to 80, or membrane-associated proteins, such as GPI-anchor proteins (if extracellular).

Such transmembrane proteins (tetraspanin or other transmembrane protein) are advantageously engineered by grafting of a so-called snorkel transmembrane segment comprising:
- an intracellular (intravesicular) linking portion, rich in basic amino acids, especially close to the transmembrane section, and linked to the transmembrane protein or fragment thereof (such as a tetraspanin) by a glycine and serine-rich linker;
- a transmembrane domain, for instance of about 20-25 aminoacids, essentially consisting of hydrophobic aminoacids (even if between one and five, preferably three, serine and/or threonine residues are tolerated, depending on the secondary structure of the transmembrane domain);
- an extracellular (extravesicular) linker.

Such a snorkel segment is advantageous for positioning the (above-described) segment for the binding of the exogenous chemical or the derivatization segment at the C-terminal part of a protein if this extremity is cytosolic in the native protein (e.g. for tetraspanins) and/or to reduce the risk of interference of the grafted segment on the normal function of the transmembrane protein.

In other words, advantageously, especially if the C-terminal part of the native transmembrane protein is cytosolic, the engineered transmembrane protein further comprises an additional transmembrane region, such as the so-called snorkel sequence (SEQ ID NO:81) and sequence sharing the same structure, preferably a sequence comprising (from N to C):
- a segment of a size between 3 and 20, preferably between 5 and 15 amino acids consisting of amino acids selected from G, S and A and 0, 1 or 2 P residues,
- at least one amino acid selected from K or R, preferably two (forming a positively-charged segment to interact with the lipid bilayer),
- a transmembrane segment,
- at least one amino acid selected from K or R (forming a second positively-charged segment to interact with the lipid bilayer) and
- a segment between 3 and 20, preferably between 5 and 15 amino acids consisting of amino acids selected from G, S and A residues.

Possibly, additional amino acids are present between these segments.

Alternatively, or in addition, the sequence sharing the same structure as the snorkel region shares an identity of at least 50%, preferably at least 60% of identity, preferably at least 70, at least 75, at least 80, at least 85, at least 90, at least 95% of identity over the full-length of SEQ ID NO:81 (thus in addition of sharing the 5 segments described above).

"EVs", preferably, refer to particles having a diameter between 30 and 500 nm. They are lipid bound vesicles secreted by cells into the extracellular space. The three main types of EVs are microvesicles (MVs), exosomes, and apoptotic bodies, a nomenclature based on their different biogenesis pathways. The EVs also differ in size, content, and function. Advantageously, EVs comprise various surface proteins, further to the tetraspanins, and/or are enriched in cholesterol and ceramide (thereby containing lipid rafts).

In the context of the present invention, one or several EV surface molecules are genetically altered to comprise a segment recognizing a chemical derivative 2 so that these surface molecules, when expressed into the eukaryotic (mammalian or plant) cells, will then sort them to EV membranes with the segment recognizing the chemical derivative 2 being exposed outside the EV, able to bind the chemical derivative 2, when present. As it will be detailed below, this offers an advantageous tool for delivering such recombinant molecule, hence the EV, to a desired *in vivo* target.

This engineered eukaryotic (mammalian or plant) cell, or cell line, will thus generate extracellular vesicles comprising an engineered transmembrane (or GPI-anchored) protein.

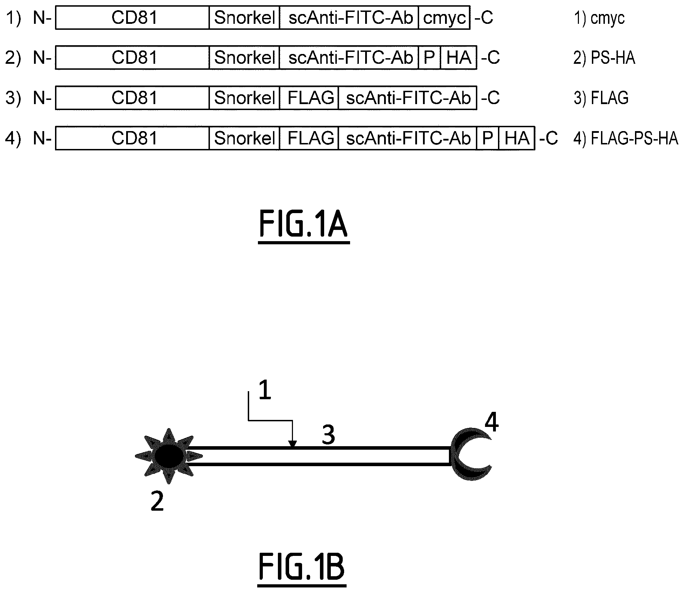

In a subsequent step, and to reference with Figure 1b, a bifunctional molecule 1 is applied to such cells or, preferably, to such extracellular vesicles, and this bifunctional molecule 1 comprises (i) the chemical derivative 2 (binding with high-affinity to the transmembrane protein present in the extracellular vesicle) and (ii) the ligand 4 to be tested, for instance *in vivo.*

In the context of the present invention, the wording "bifunctional molecule (1)" means an artificial molecule designed to bind the engineered surface protein of the EV and a cellular target. In the context of the present invention, the wording "bifunctional", is meant to be open to other functions and means two or more functions, for instance (see below), when the chemical derivative 2 is a fluorescent moiety, a third function is present (or might be considered to be present), yet such molecule falls under the scope of the present invention. Similarly, a "bifunctional molecule" comprising several binding ligands 4 (same or different) also falls under the scope of the present invention. Advantageously, the bifunctional molecule 1 comprises a linking region 3, preferably a peptide.

Advantageously, the chemical derivative 2, such as fluorescein and its derivatives, is arranged, via the linking region 3, in the bifunctional molecule 1 at a position not interfering with the binding of the ligand 4 to its desired target.

The bifunctional molecule 1 is preferably a (ribosomal) peptide or a (ribosomal) peptide derivatized with the chemical derivative 2; in other words, preferably, the ligand 4 is a (ribosomal) peptide, which advantageously further comprises a linking region 3 and a chemical derivative 2.

Preferred peptides or peptides derivatized with the chemical derivative 2 are:
- antibodies (monoclonal or polyclonal or synthetic) or fragments thereof or nanobodies or fragments thereof,
- solubilized receptors and fragments thereof(acting as decoy),
- ligands of receptors,
- cytokines, enzymes, peptidic hormones,
- glycoproteins.

Other preferred biomolecules comprise a non-peptidic segment or are fully non-peptidic or non-ribosomal peptides:
- ramified or segmented oligosaccharides,
- non-ribosomal peptides, such as antibiotics, cyclopeptides, lipopeptides,
- aptamers.

Advantageously, the aptamer can harbor the function of the chemical derivative 2 and/or of the ligand 4.

Alternatively, preferred aptamers are further derivatized with fluorescein (fluorescein isothiocyanate; FITC) as chemical derivative) and harbor the function of the ligand 4.

Fluorescein isothiocyanate (FITC) is a preferred chemical derivative 2. In this specific setting, the transmembrane protein of the extracellular vesicle (exosome) advantageously comprises an antibody fragment, such as a single chain antibody, having a very high affinity for fluorescein.

A related aspect of the present invention is an engineered eukaryotic (mammalian or plant) cell expressing an engineered transmembrane protein (or an engineered GPI anchor protein) having the potential to be sorted into an extracellular vesicle (exosome) from the said eukaryotic (mammalian or plant) cell, this transmembrane protein comprising at least one extracellular domain, wherein the extracellular domain of the said transmembrane protein comprises a derivatization segment (or the GPI anchor protein is located at the extracellular layer of the cell membrane and comprises a derivatization segment).

Preferably, such derivatization segment is compatible with grafting of elements through copper-free "click chemistry", for instance in generating *in situ* exposed azide groups and/or based on a derivatization segment comprising specific sugar residues at the EV (or even at the cell) surface.

This eukaryotic cell is preferably a mammalian mesenchymal stem cell, preferably a Human mesenchymal stem cell, and/or an immortalized cell line, such as HEK293 cells.

Advantageously, this cell (plant, yeast or mammalian, Human) will generate extracellular vesicles on which a ligand is to be grafted, which has been validated (preferably using the cell of the first aspect described here above, or using the method described below) for its capacity to correctly target the exosome, preferably *in vivo.*

Hence, a related aspect of the present invention are the EVs obtainable from the above engineered Eukaryotic cells, such as mammalian (Human) mesenchymal stem cells.

Another related aspect of the present invention is a method to select a targeting ligand for an extracellular vesicle (exosome) comprising the step of:
- selecting an engineered Eukaryotic (plant, mammalian, Human) cell line (or the cell line described here above) expressing a transmembrane protein, or a GPI anchor protein (exposed to the external layer), having the potential to be sorted into an EV from the said Eukaryotic cell, the said transmembrane protein comprising one or more extracellular domain(s), or of the GPI anchor protein comprising an extracellular domain, wherein the extracellular domain of the said transmembrane protein or of the said GPI protein, of the said engineered Eukaryotic cell comprises a segment having a high affinity for a chemical derivative not expressed by the said Eukaryotic cell;
- growing cells from the said engineered Eukaryotic cell line;
- obtaining EV from the said grown cells and
- applying to the transmembrane protein, or to the GPI anchor protein, of the obtained EV a bifunctional molecule 1, the first function being the ligand 4 to a desired target (preferably a tissue or a cell type of a patient), and the second function being the said chemical derivative 2, so that the said chemical derivative 2 is bound with high-affinity to the said transmembrane protein, or to the said GPI anchor protein, and the ligand 4 is still able to recognize its desired target;
- measuring, *in vitro* and/or *in vivo,* the binding of the said EV to the said target, or a preferential accumulation of the targeted EV in the vicinity of such target (e.g. accumulation in a specific tissue)
- determining which ligand allows the correct targeting of the EV, preferably taking into consideration (i) the on-target proportion of EV and/or (ii) the off-target proportion of EV.

Even if the binding of the chemical derivative 2, being part of the bifunctional molecule 1, to the transmembrane protein is not covalent, thus potentially lost upon the *in vivo* journey of the extracellular vesicle (exosome), the inventors have found that such approach is valuable and allows to efficiently screen for ligands-induced *in vivo* targeting of EV.

Indeed, if the affinity is high enough, the inventors have found that the chemical derivative 2 remains attached to the transmembrane protein for a time period long enough (see below for the definition of the affinity and dissociation constant).

A fluorescent chemical derivative 2 is advantageous. Among them, fluorescein (FITC) is preferred as allowing both a high-affinity interaction with known specific antibodies and a direct fluorescent readout.

Other molecules 2 able to bind to chemical derivatives with high affinity are also preferred. Among them is avidin (or neutravidin or streptavidin or other avidin derivatives). In that case, the chemical derivative of the present invention will be a vitamin (biotin), yet the bifunctional molecule remains an artificial construction, such as a biotinylated ligand. Trypsin and BPTI can also be chosen, with the advantage of the chemical derivative (BPTI) being a peptide; hence potentially allowing a pure peptidic nature for the bifunctional molecule 1. It is to be noted that, even if biotin may be present in the cell culture medium, or even (accidentally) within the cells or EV thereof, in the context of the present invention, biotin, or similar molecules not actively expressed (produced, fermented) by the cell, is considered as a potential chemical derivative 2.

Advantageously, the bifunctional molecule 1 is a peptide, further derivatized with the second function 2 (the chemical derivative, such as fluorescein), preferably the peptide being an antibody or a fragment thereof.

Other structures for the ligand part 4 of the bifunctional molecule 1 can be contemplated, such as peptides being different of antibodies, such as peptidic ligands or cytokines or hormones, as well as sugar residues (e.g. ramified or segmented oligosaccharides), known to be fixed by a specific cell type, or preferentially accumulated in a specific type of tissue or aptamers.

In the context of the present invention, in association with the bifunctional molecule and/or in the above selection method, the wording "high affinity" preferably refers to a dissociation constant (Kd) value of the chemical derivative and/or of the second function bound to the transmembrane protein (or GPI anchor protein) in the nano- to femtomolar range, meaning lower than 10⁻⁷, preferably lower than 10⁻⁸, preferably lower than 10⁻⁹, preferably lower than 10⁻¹⁰, preferably lower than 10⁻¹¹, preferably lower than 10⁻¹² Molar (at 25°C), such as monovalent antibodies having an affinity in the femtomolar range (e.g. about 50 fM).

Alternatively, or in addition, in the context of the present invention, "high-affinity" preferably refers to a chemical derivative dissociating from the transmembrane molecule at a rate lower than 10⁻⁵ s⁻¹, preferably lower than 10⁻⁶ s⁻¹, or even close or lower than 10⁻⁷ s⁻¹. Preferably, the dissociation is measured as in Boder et al., PNAS, 2000, Vol. 97, pages 10701-10705.

Preferably, in the above method, the transmembrane protein is a tetraspanin (CD9, CD63, CD37, CD81, or CD82).

A related aspect of the present invention is a targeting ligand obtainable by the selection method as described above.

Possibly such targeting ligand is artificial: either a specific chemical or a non-naturally occurring biomolecule, such as new peptide sequence, new oligosaccharide association, or ramification, ...

Preferably, this targeting ligand is covalently grafted on an EV, such as on an EV obtained as above, preferably an EV produced from (human) mesenchymal stem cells and/or from an (adherent) immortalized (human) cell line.

Such covalent grafting is advantageously achieved through a chemical condensation with the extracellular (or GPI anchor) protein described here above.

The inventors do consider that the (chemical) grafting of a ligand on a transmembrane (GPI anchor) protein from an extracellular vesicle is easier to achieve, in practice, than the (genetic) engineering of specific cells, even if the second approach appears to be more straightforward.

However, as an alternative, if the ligand is a (ribosomal) peptide (such as an antibody fragment recognizing a cellular target, or a ligand for cell receptors), the transmembrane (or GPI anchor) protein can advantageously be engineered as a fusion protein with such peptide ligand, then expressed, advantageously transiently, in a cell for EV production, preferably (Human) mesenchymal stem cells and/or from an (adherent) immortalized (Human) cell line.

In other words, when the targeting ligand is a peptide, another aspect of the present invention is an engineered Eukaryotic (mammalian, plant) cell expressing a transmembrane (or GPI anchor) protein having the potential to be sorted into EV from the said Eukaryotic (mammalian, plant) cell, wherein the extracellular domain of the said transmembrane protein comprises the targeting ligand as identified above.

A variant of the above-method is a method to produce targeting EVs comprising the step of:
- selecting an engineered Eukaryotic (plant, mammalian, Human) cell line (or the cell line described here above) expressing a transmembrane protein, or a GPI anchor protein (exposed to the external layer), having the potential to be sorted into an EV from the said Eukaryotic (plant, mammalian, Human) cell, the said transmembrane protein comprising one or more extracellular domain(s), or of the GPI anchor protein comprising an extracellular domain, wherein the extracellular domain of the said transmembrane protein or of the said GPI protein, of the said engineered Eukaryotic (plant, mammalian, Human) cell comprises a segment having a high affinity for a chemical derivative not expressed by the said Eukaryotic (plant, mammalian, Human) cell;
- growing cells from the said engineered Eukaryotic cell line;
- obtaining EV from the said grown cells and
- applying to the transmembrane protein, or to the GPI anchor protein, of the obtained EV a bifunctional molecule 1, the first function being the ligand 4 to a desired target (preferably a tissue or a cell type of a patient), and the second function being the said chemical derivative 2, so that the said chemical derivative 2 is bound with high-affinity to the said transmembrane protein, or to the said GPI anchor protein, and the ligand 4 is still able to recognize its desired target;
- measuring, *in vitro* and/or *in vivo,* the binding of the said EV to the said target, or a preferential accumulation of the targeted EV in the vicinity of such target (e.g. accumulation in a specific tissue);
- determining which ligand allows the correct targeting of the EV, preferably taking into consideration (i) the on-target proportion of EV and/or (ii) the off-target proportion of EV;
- obtaining EVs from Eukaryotic (plant, mammalian, Human) cells;
- grafting the identified ligand 4 on the said EVs
- Isolating or formulating these grafted EVs.

Advantageously, the grafting step is achieved using copper-free click chemistry.

Another related aspect of the present invention is a pharmaceutical composition comprising such extracellular vesicle with a grafted, or engineered, transmembrane (or GPI anchor) protein.

### Examples.-

### Example 1 - the nucleic acid constructs

The inventors have generated four nucleic acid molecules, all comprising the CD81 tetraspanin and a so-called snorkel region to generate an extracellular C-terminal part.

Two nucleic acids have been generated to encode a FLAG cassette, and two do not encode such FLAG cassette, all the four nucleic acid molecule further comprising a high-affinity single-chain variable fragment against FITC, followed either by a c-Myc tag, or an HA tag after a PS region (also a site specifically recognized by PreScission protease) or no C-terminal additional tag (see Fig. 1a).

### Example 2 - in vitro validations

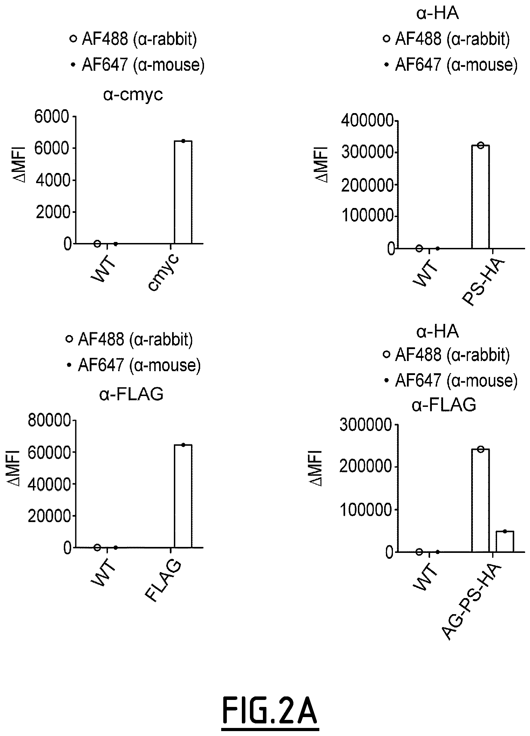

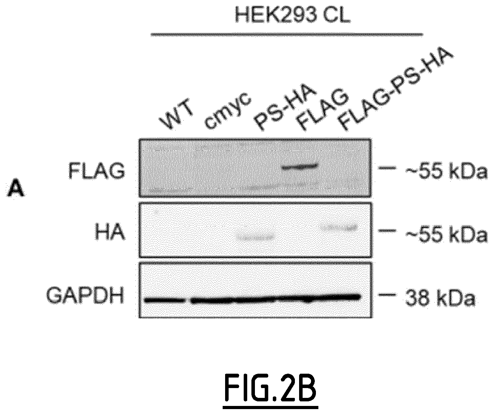

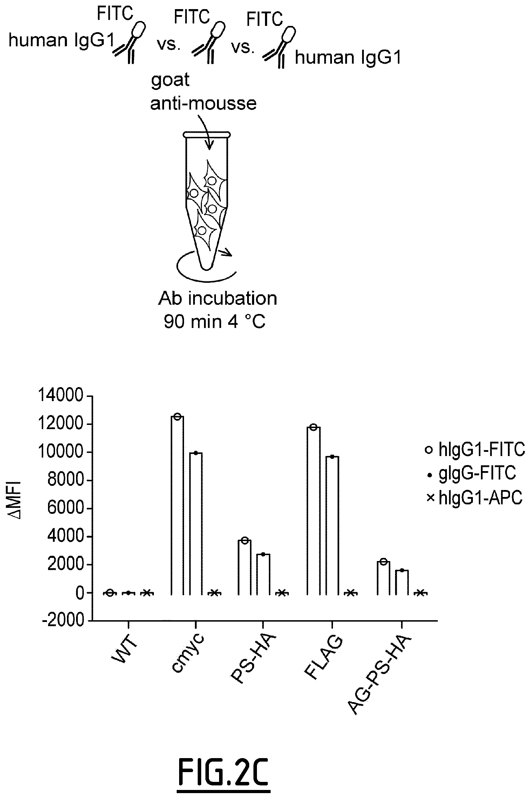

The inventors have performed flow cytometry experiments for the transfected cells, using anti-cMyc, anti-FLAG, anti-HA antibodies. As shown in Figure 2A, there was a specific signal for all the tags that have been inserted, meaning that the Tags have been correctly expressed and that the 3D structure of the engineered protein allows still a specific recognition. A Western blot control also shows correct expression and no cross-reactivity (Fig. 2B).

The addition of a FITC-antibody to these cells resulted into a specific binding to the expressed anti FITC fragment, being part of the engineered protein, again meaning a correct expression and a correct 3D folding of the recombinant protein.

### Example 3 - EV production and characterization

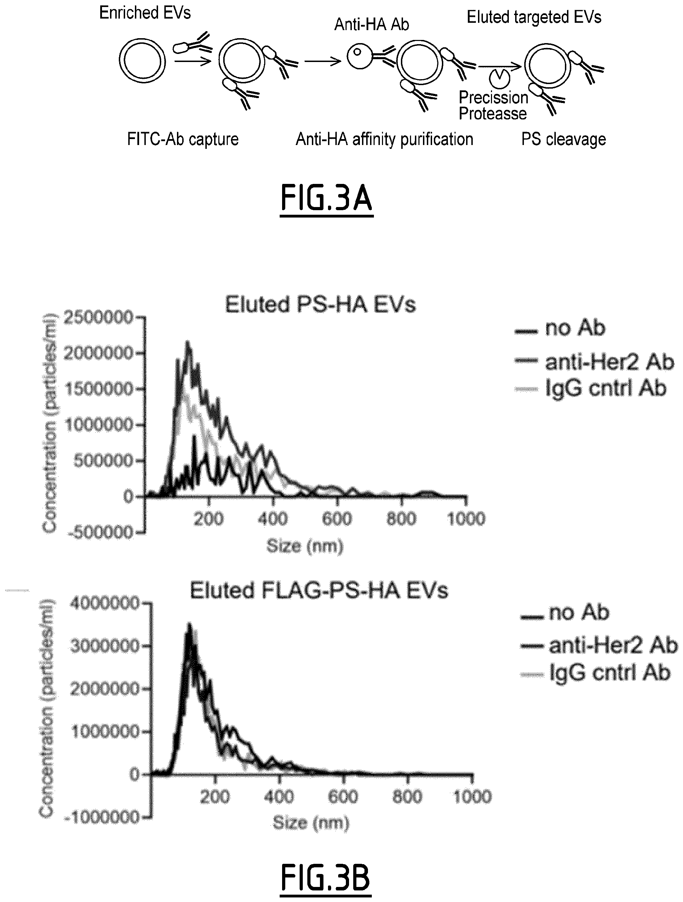

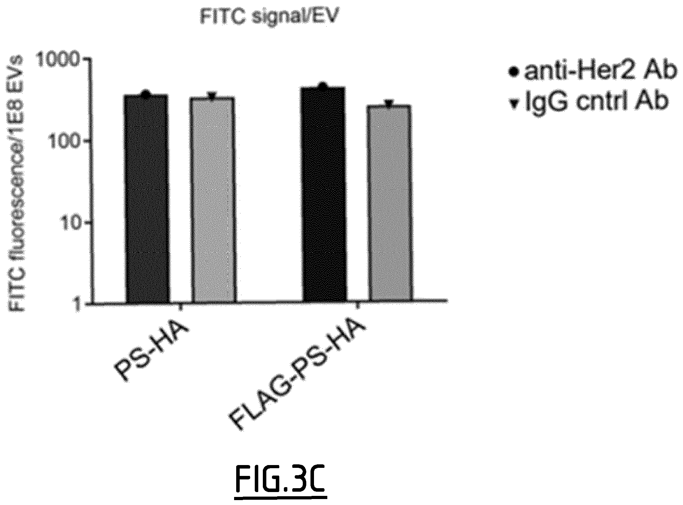

EVs are produced from HEK293 cells, then purified using magnetic beads coupled to anti-HA antibodies followed by cleavage with PreScission Protease (FIG. 3A). Similar EV sizes have been measured (Fig. 3B). The EVs have also been marked with FITC-antibodies and the fluorescence has been measured (Fig. 3C.).

### Example 4 - functional recognition of the target in vitro.

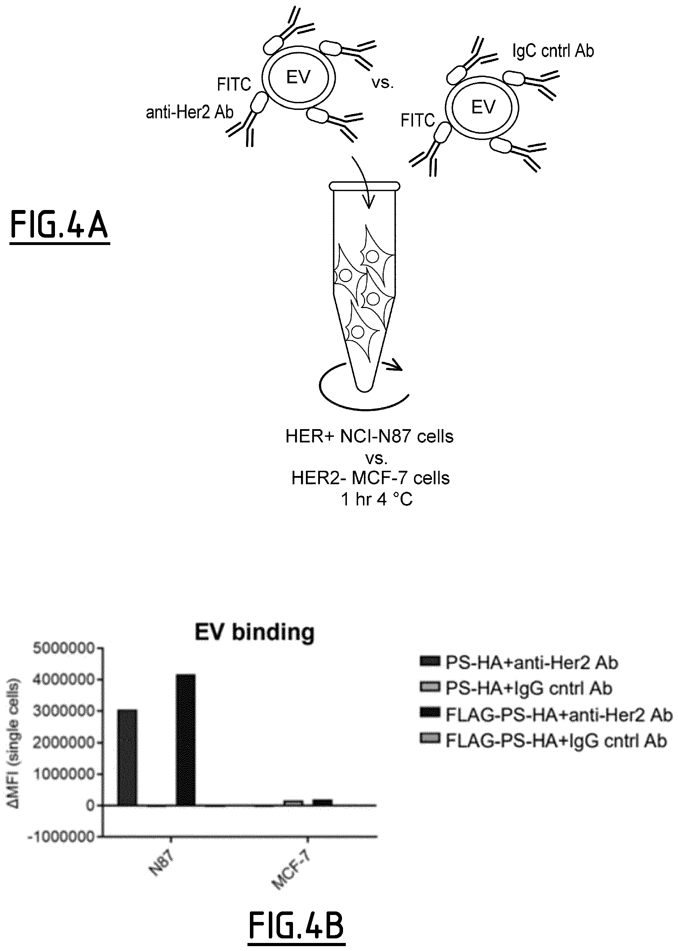

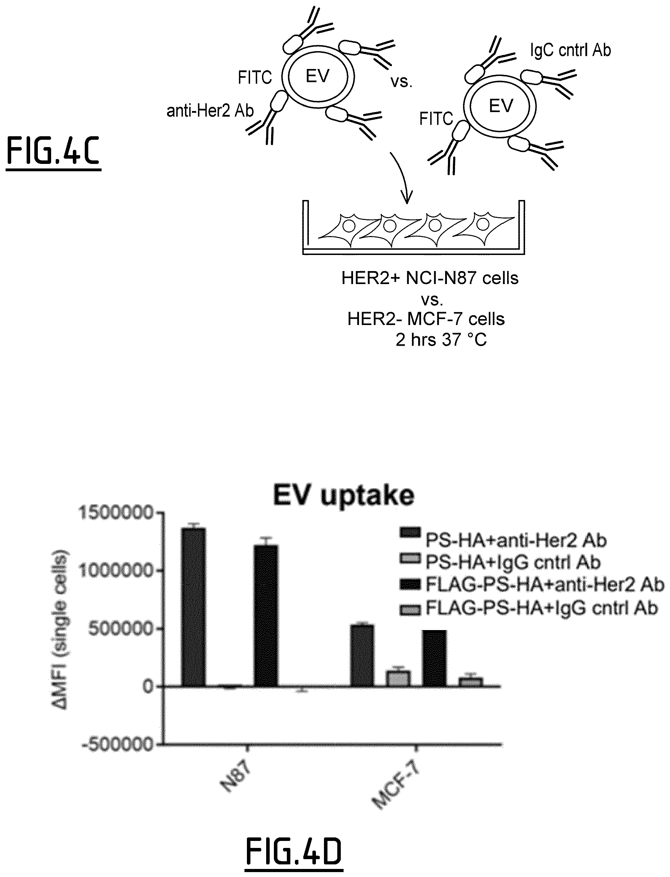

EVs are produced, then marked with FITC-anti Her2 or FITC-control antibodies before anti-HA affinity purification and elution. These labelled EVs are then mixed with NCI-N87 cells or with MCF-7 cells (Fig. 4A). As shown in Figure 4B, a marked and specific association between the anti-Her2-EVs and the NCI-N87 cells have been obtained and only a limited association to MCF-7 cells. The inventors have then measured the uptake of FITC-anti-Her2-EV by (HER2+) NCI-N87 cells or by (HER2-) MCF-7 cells (Figures 4C and D).

### Example 5 - constructs based on other tetraspanins

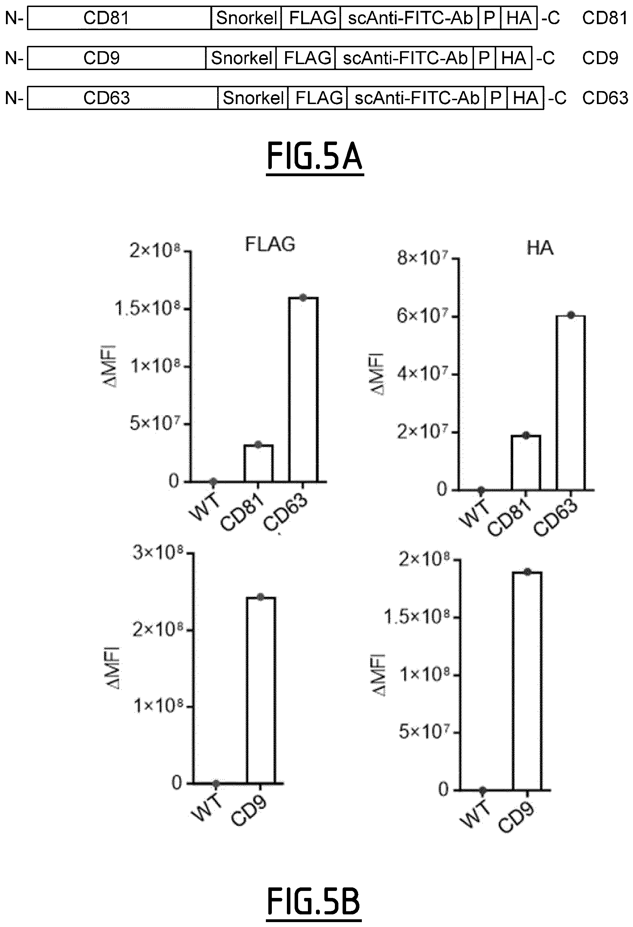

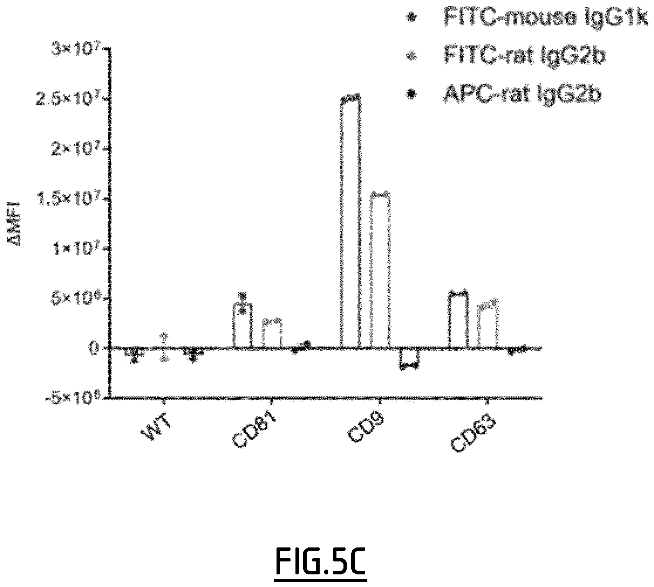

The inventors have then generated similar construct, but with CD9 or CD63, in replacement of CD81. See the constructs at Figure 5A. These recombinant proteins were expressed (Fig. 5B) and able to capture the FITC-antibodies (Fig 5C).

## Claims

1. An engineered Eukaryotic cell expressing an engineered transmembrane protein having the potential to be sorted into an extracellular vesicle (EV) from the said Eukaryotic cell,
the said engineered transmembrane protein harboring one or several extracellular domains, wherein at least one of the extracellular domain of the said engineered transmembrane protein comprises a segment having a high affinity for a chemical derivative (2) not expressed by the said Eukaryotic cell.

2. An extracellular vesicle obtainable from the engineered Eukaryotic cell of claim 1, preferably wherein the Eukaryotic cell of claim 1 is a mammalian mesenchymal stem cell, more preferably a human mesenchymal stem cell.

3. An engineered Eukaryotic cell expressing an engineered transmembrane protein having the potential to be sorted into an extracellular vesicle from the said Eukaryotic cell, the said engineered transmembrane protein harboring one or several extracellular domains, wherein at least one of the extracellular domains of the said engineered transmembrane protein comprises a derivatization domain, preferably the said engineered transmembrane protein being a tetraspanin engineered to comprise a fifth transmembrane region, such as a sequence sharing at least 50% of identity with SEQ ID NO:81 .

4. An extracellular vesicle obtainable from the engineered Eukaryotic cell of claim 3, preferably wherein the Eukaryotic cell of claim 3 is a mammalian mesenchymal stem cell, more preferably a human mesenchymal stem cell.

5. The engineered Eukaryotic cell or extracellular vesicle according to any one of the preceding claims wherein the engineered transmembrane protein is selected from SEQ ID NO:1 to 80 or comprises a tetraspanin (CD9, CD63, CD37, CD81, or CD82), or a functional fragment thereof, or a protein sharing at least 95% of identity over the full-length of the said SEQ ID NO:1 to 80, of over the full-length the tetraspanin, or of the functional fragment thereof.

6. A method to select a ligand (4) targeting an extracellular vesicle to a specific cell, organ, or tissue comprising the steps of:
- selecting an engineered Eukaryotic cell line expressing an engineered transmembrane protein having the potential to be sorted into an extracellular vesicle from the said Eukaryotic cell, the said transmembrane protein comprising at least one extracellular domain, wherein at least one of the extracellular domain of the said transmembrane protein of the said engineered Eukaryotic cell comprises a segment having a high affinity for a chemical derivative (2) not expressed by the said Eukaryotic cell;
- growing cells from the said engineered Eukaryotic cell line;
- obtaining extracellular vesicles from the said grown cells and
- applying to the transmembrane protein of the obtained extracellular vesicle a bifunctional molecule (1),
the first function being the ligand (4) to a desired target, and the second function being the said chemical derivative (2), so that the said chemical derivative (2) is bound with high-affinity to the said transmembrane protein and the ligand (4) is still able to recognize its desired target;
- measuring, *in vitro* and/or *in vivo,* the binding of the said extracellular vesicle to the said target.

7. The method of claim 6, wherein the chemical derivative (2) is selected from the group consisting of biotin and fluorescein.

8. The method of claim 6 or 7, wherein the bifunctional molecule (1) is a peptide (3, 4) derivatized with the second function (2), preferably the peptide (3, 4) being an antibody or a fragment thereof.

9. The method according to any one of the preceding claims 6 to 8, wherein the high affinity is a dissociation constant (Kd) value of the chemical derivative (2), and/or of the second function bound to the transmembrane protein, lower than 10⁻⁹, preferably lower than 10⁻¹² Molar (at 25°C).

10. The method according to any one of the preceding claims 6 to 9, wherein the engineered transmembrane protein is selected from SEQ ID NO:1 to 80, or comprises a tetraspanin (CD9, CD63, CD37, CD81, or CD82), or a functional fragment thereof, or a protein sharing at least 95% of identity over the full-length of the said SEQ ID NO:1 to 80, over the full-length of the tetraspanin, or a functional fragment thereof.

11. A targeting ligand (4) obtainable by the method according to any one of the preceding claims 6 to 10.

12. The targeting ligand (4) of claim 11 being covalently grafted on the extracellular vesicle of claim 4.

13. The targeting ligand (4) of claim 11 being non-covalently bound on the extracellular vesicle of claim 2.

14. An engineered Eukaryotic cell transiently expressing an engineered a transmembrane protein having the potential to be sorted into an extracellular vesicle from the said Eukaryotic cell, the said engineered transmembrane protein comprising one or several extracellular domains, wherein at least one of the extracellular domain of the said transmembrane protein comprises the targeting ligand (4) of claim 11.

15. A pharmaceutical composition comprising the grafted extracellular vesicle of claim 12 or 13, or extracellular vesicles obtained from the engineered cells of claim 14.
